# EUROPEAN PATENT APPLICATION

(11) **EP 2 281 593 A1**
(43) Date of publication of application: **09.02.2011**
(21) Application number: 09164984.8
(22) Date of filing: 09.07.2009
(51) Int. Cl.: A61M 16/00

(54) **Medical blower control via pneumatic patient model**

(71) Applicant: Dräger Medical GmbH, 23558 Lübeck (DE)
(72) Inventor: Herben, Eugène, 5672 SC Nuenen (NL); Schott, René, 41063 Mönchengladbach (DE); Robben, Koen, 5046 RK Tilburg (NL)
(74) Representative: UEXKÜLL & STOLBERG

(57) **Abstract**

A medical ventilator, having a blower (412), is controlled in operational use through a mathematical model of the ventilator-patient combination in order to control the airflow rate via controlling the pressure. Time delay control and iterative learning can be applied in order to improve accuracy of the control.

## Description

### FIELD OF THE INVENTION

The invention relates to medical equipment comprising a medical ventilator and a control system for controlling operation of the ventilator in operational use.

### BACKGROUND ART

Medical ventilators (or: resuscitators) are often based on a system with a ducted mechanical fan, also referred to as a "blower". The blower used in such medical equipment is an actuator, driven by an electric motor and designed for accurate control of air pressure within a system, e.g., a patient. The desired magnitude of the pressure is a function of the motor speed or duty-cycle of the control signal for the motor. This desired magnitude of the pressure is relatively independent of the system in which the pressure is to be controlled. An example of such a blower is the radial blower also referred to as a centrifugal fan.

The flow rate (typically: liters per minute), at which a blower supplies air or another respiratory gas to the system, depends on the resistance to the flow (also referred to as "static pressure" or "system pressure") that the blower has to overcome. A specific value of the flow rate corresponds to a specific value of the resistance for a specific fan speed (revolutions per minute). A particular type of blower is characterized by a set of curves in a Cartesian space spanned by a first axis, each particular point whereof corresponds to a particular value of the flow rate, and a second axis, of which each particular point corresponds to a particular value of the resistance . Each specific one of the curves corresponds to a specific value of the fan speed. For, e.g., a radial blower, the curves are largely uniform, and offset with regard to one another in the direction of the second axis. For additional technical information see, e.g., "Fan Fundamentals: Fan Selection, Application-Based Selection, Performance Theory", Rev 2, June 2005, Greenheck Fan Corp.

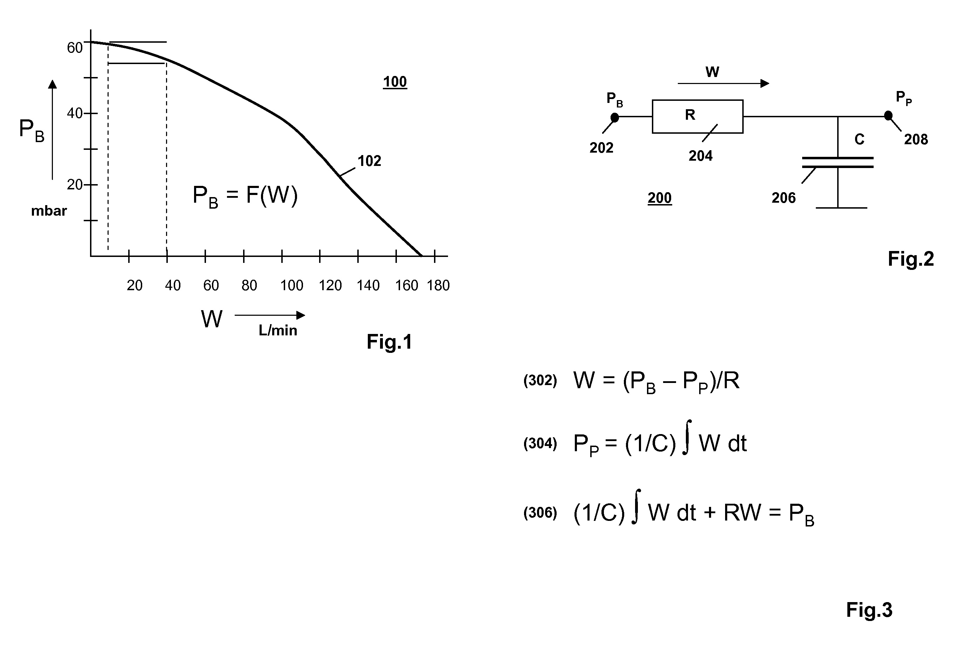

The blower is not an ideal pressure source, because the flow rate decreases with increasing system pressure (or: resistance to the flow). As a result, the flow rate is sensitive to fluctuations in the system pressure. Fig.1 is a diagram 100 of a typical fan curve 102 illustrating this sensitivity. Curve 100 represents the dependence of the flow rate (horizontally, in liters per minute) on the system pressure (vertically, in mbar) at a particular fan speed. In principle, the blower can be used to control the flow rate, but the flow rate varies with varying system pressures. For example, if the system pressure varies between 55 mbar and 60 mbar, the flow rate fluctuates between 5 l/sec and 40 l/sec in the example illustrated.

In medical ventilators, the system pressure (flow resistance) varies significantly during ventilation. As a result, it is difficult to control the flow rate if the magnitudes of the current flow rate and of expected system pressures are not known in advance with a good enough accuracy. The current system pressure can be measured and be used in a feedback control loop to control the blower via its electronic control circuitry. However, the system pressure changes in dependence on the actual flow rate, and the work point of the blower will change as well, responding to the fluctuating system pressure. This will cause instabilities in the medical ventilator, as a result of limits to the accuracy of the pressure sensor, the dynamic behavior of the sensor, etc., which in turn lead to unstable and inaccurate flow rate control.

Various systems are known in the art that control the flow. Conventionally, the gas flow rate is controlled by actuation of a gas flow valve. Together with a combination of a feed-forward flow control gain component and/or a feedback error correction (e.g., a proportional, integral and derivative error feedback control), this gives rise to the required response.

Another known method to control the gas flow rate is to make explicit use of the properties of the blower. Controllably varying the speed of the blower can be used to control the flow, based on the predetermined relationship between the system pressure and the flow rate, as discussed with reference to Fig.1. The blower is designed to respond quickly to a change in inspiration or expiration by means of minimizing its inertia. In this case, a feedback controller can be used as well for control of the gas flow. However, variations in the system pressure can change the flow rate, even at a constant blower speed. This problem cannot fully be solved with feedback control. The continuously changing system pressure usually leads to an unstable system or oscillations around the target flow.

US 20060065270 discloses a system and method for controlling the flow of gas from a medical ventilator into a patient's lungs. The control system provides for a non-linear feed-forward controller to correct for disturbances caused by back pressure at the outlet of the blower of the medical ventilator. For this purpose, a pressure transducer is provided to measure the back pressure. Additionally, the known system allows for a feedback controller to correct for the differences between the rate of the actual gas flow and the targeted gas flow rate. For this purpose a flow rate transducer is provided. The control system may account for each of the gas flow rate error and the back pressure disturbance to provide for a quick and accurate adjustment to achieve the targeted gas flow rate. However, this known approach does not solve the instabilities occurring at low flow rates

### SUMMARY OF THE INVENTION

A blower can be considered an actuator for controlling pressure, whereas, in above applications, the blower is used to control the flow rate. A blower is therefore the incorrect actuator if the physical quantity to be controlled is the flow rate.

In order to be able to still use a blower for accurate control of the flow rate, the inventor proposes to employ a mathematical model of the ventilator-patient combination in operational use of the blower. The mathematical model provides a relationship between the physical quantity that can be accurately controlled, namely, the pressure, and the physical quantity that needs to be accurately controlled, namely the flow rate. The model enables to regulate the blower, i.e., a pressure actuator, through adjusting the physical quantity most suitable for the blower, i.e., the pressure, to thereby control the desired quantity, i.e., the flow rate.

More specifically, the invention relates to medical equipment comprising a medical ventilator, comprising a blower, and a control system for controlling operation of the ventilator. The control system comprises an input for receiving a flow rate set-point representative of a first target value of a flow rate. The control system further has first means coupled to the input for receipt of the flow rate set-point and configured for determining a pressure set-point, representative of a second target value of a pressure, based on a mathematical model of the ventilator in combination with a patient. The model includes a patient parameter indicative of a pneumatic impedance of the patient. Examples of the pneumatic impedance include, e.g., a resistance value representative of a resistance of airways of the patient, and a compliance value representative of a compliance of a lung-thorax subsystem of the patient. The control system also has second means, coupled to the input for receiving the flow rate set-point, and coupled to the first means for receiving the pressure set-point. The second means is configured for determining a blower set-point, based on predetermined fan curves of the blower, for control of the blower.

The blower set-point in the invention does not depend directly on the actual pressure as measured, but uses a system model that predicts the tendency of the system pressure as a function of the required flow rate set-point. The system does not use a possibly unstable actual pressure value. Instead, the invention uses a stable predicted pressure set-point and a mathematical model, e.g., a simple compliance-resistance model, which predicts the evolution of the system pressure with respect to the required flow rate set-point. In the invention, the parameters for feed-forward control are determined by the ventilation characteristics of the patient or, more specifically, by the resistance and the compliance of the patient. In a first order approximation, these characteristics can be simulated by means of a simple linear model. A more complex model can be used, but does not change the operating principle of the invention. From this patient model the parameters for the feed-forward control are determined.

The medical equipment of above configuration performs well if the model is a truthful representation of reality. However, the used model that specifies the interrelationship between the various physical quantities, or the used values of the model's parameters may not be accurate enough for practical purposes, in the sense that the blower's control may not be optimal. Therefore, it is proposed to use one or more algorithms to generate an estimate of an error in the model, using current information captured by one or more sensors, here a pressure sensor and a flow rate sensor, and to adjust control of the blower accordingly. Examples of known algorithms that can be applied as control strategies, individually or in combination, are "Time Delay Control" (TDC) and "Iterative Learning Control" (ILC).

The TDC strategy employs past observation of the system response and control inputs to directly modify the control actions rather than adjusting the controller gains. It updates its observation of the system every sampling period. See, e.g., "A Time Delay Controller for Systems with Unknown Dynamics", Yousef-Toumi, K., et al., Journal of Dynamic Systems, Measurement and Control, Mar. 1990, Vol. 112, pp.133 -142.

The ILC strategy tracks control for a system that works in a repetitive mode. By using information from previous repetitions a suitable control action can be found iteratively. See, e.g., Arimoto, S., et al., (1984); "Bettering operation of dynamic systems by learning: A new control theory for servomechanism or mechatronic systems"; Proceedings of 23rd IEEE Conference on Decision and Control, Las Vegas, NV. pp. 1064 -1069, and Kawamura, S., F. Miyazaki and S. Arimoto (1985); "Applications of learning method for dynamic control of robot manipulators"; Proceedings of the 24th IEEE Conference on Decision and Control, Ft. Lauderdale, FL, pp. 1381-1386. As the ventilation of the patient follows a cyclic temporal pattern, ILC can be used to advantage.

Accordingly, in an embodiment of the medical equipment in the invention, the control system further comprises a flow rate sensor for sensing a current flow rate value of a flow rate at an output of the blower; and a pressure sensor for sensing a current pressure value of a pressure at an output of the blower. The control system further has third means, coupled to the flow rate sensor and the pressure sensor, for receiving the current flow rate value and the current pressure value. The third means is configured for determining a correction signal for correction of the blower set-point. The correction signal is determined using at least one of the following strategies: a time delay control algorithm, an iterative learning control algorithm, introduced above. As an option, the control system in the medical equipment of the invention has a selection mechanism in order to select between the time delay control algorithm on the one hand, and the iterative learning control algorithm on the other hand. The time-delay control mechanism is preferred if the patient is under narcosis, i.e., if the patient's reflexes have been artificially suppressed and the patient remains more or less in the same physiological state. On the other hand, the iterative learning control algorithm is more suitable if the patient's physiological state changes, e.g., due to his/her body reflexes, as this algorithm automatically adapts to the changes. The selection mechanism uses a manual selection input, such as a switch or dial, at the user-interface of the medical equipment. Alternatively, the selection mechanism is controlled via an external source, e.g., a therapy-driven expert-system that determines which algorithm to exploit based on diagnostics-related parameters.

In a further embodiment, the mathematical model is a linear model, wherein the value of the pneumatic impedance is chosen independent of the flow rate and the pressure.

The invention also relates to a control system for controlling operation of medical equipment comprising a medical ventilator with a blower, as specified above. Preferably, the control system is user programmable so as to be configurable for control of any of a variety of medical ventilation devices.

The first, second, third means are implemented, e.g., using signal processing software being executed on a data processing system such as a general-purpose computer, or dedicated electronic signal processing circuitry, or a combination thereof.

### BRIEF DESCRIPTION OF THE DRAWING

The invention is explained in further detail, by way of example and with reference to the accompanying drawing, wherein:
Fig.1 is a diagram for illustrating a fan curve;
Fig.2 is a diagram illustrating a model of a system with a patient and a blower in operational use;
Fig.3 includes mathematical expressions explaining the model of Fig.2;
Fig.4 is a block diagram of a system in the invention; and
Fig.5 includes mathematical expressions explaining the system of Fig.4.

Throughout the Figures, similar or corresponding features are indicated by same reference numerals.

### DETAILED EMBODIMENTS

The gas flow rate of a medical ventilator can be controlled through the use of a variable speed blower or a fan. The speed of such a blower can be rapidly increased or decreased to impart a desired rate of flow, allowing flexibility in controlling each inspiration and exhalation. The rapid rate of change in the gas flow allows the ventilator to vary the rate of flow within the time span of a single breath cycle. Such a ventilator can gently respond to the patient's initiation of inspiration and exhalation, making rapid and repeated adjustment in order to respond accordingly.

The relationship between the system pressure and the flow rate as presented in Fig.1, discussed above, is determined by the specific configuration of the blower used. As explained above, it is difficult to control the flow rate if the values of the current system pressure and the expected system pressure are not known with high enough accuracy. The system pressure changes in response to changes in the actual flow rate, and the blower set-point (or: target value) responds to the fluctuating system pressure. Therefore, the complete system of blower interacting with the patient is such that instabilities, or at least unacceptable oscillations, may occur. These instabilities are due to, among other things, the finite response times of the components in the control mechanism, and limited accuracy of the values of the physical quantities (pressure, flow rate) being monitored.

The invention described here is based on the insight that the blower-patient system can be modeled, and that this model can be used to control the flow rate via regulating the pressure.

The blower set-point (target value) in the invention does not depend on the actual system pressure, but uses a mathematical model that predicts the tendency or evolution of the system pressure as a function of the required flow rate set-point. The system does not use a possibly unstable actual pressure, but a stable predicted pressure set-point based on the model used. The tendency aspect is obtained by using a simple Compliance-Resistance model that predicts the system pressure relative to the required flow rate set-point. In the invention, the parameters for the feed-forward control mechanism are determined by the mechanical characteristics of the patient, more specifically by the resistance and the compliance of the patient's body. The expression "resistance of the patient's body" as used within this context, and indicated by the letter "R" refers to the impedance or load, sensed by the blower when supplying a respiratory gas to the patient. Within the field of respiratory physiology, the term "compliance", indicated by the letter "C", refers to the ratio of the change ΔV in the volume V of a hollow organ as a result of a change Δp in the pressure p exerted on the wall(s) of that organ.

In a first order approximation, the mechanics of the patient can be simulated by a simple linear model. A more complex model does not change the essence of the basic approach presented here. From this patient model, the parameters for the feed-forward control are determined. After each breath, the parameter values can be updated, e.g., using iterative learning control (ILC), in order to correct for the observed deviations in the system pressure and flow rate from the desired system pressure and flow rate.

Fig.2 is a diagram of an example of a model 200 used in the invention to model the behavior of the patient when connected to a medical blower. Node 202 represents the input to the patient connected to the blower. The blower delivers an output pressure P at node 202. The induced airflow W has to overcome a resistance 204, the value R of which is determined by the combination of the resistance Rᵥₑₙₜ in the ventilator and tubing, and the resistance Rₚₐₜ in the patient's airways. Compliance 206 is the compliance C of the patient's lung-thorax system. The values of C and R are assumed constant for all practical purposes in the invention. The patient's lung-thorax system at node 208 experiences a pressure Pₚ.

Fig.3 is a set of formulae explaining model 200. An airflow W (in liters per minute) develops if there is a difference between the blower pressure P_{B} and the pressure P_{P} of the patient's lung-thorax system. The relationship between the pressure difference between nodes 202 and 208, the airflow W and the resistance R is given by expression (302). Compliance 206 acts as a buffer to airflow W, and its value C determines the pressure P_{P} at node 208 according to expression (304). Combining expressions (302) and (304) gives expression (306).

If a specific flow rate set-point (or: target value for the flow rate) W is to be achieved and the blower is perfectly controllable with regard to the required pressure set-point and the model accurately represents the behavior of the blower-patient combination, the pressure set-point of the controller for the blower is given by expression (306). Resistance R contains contributions from the resistance Rᵥₑₙₜ within the ventilator and tubing, and from the resistance Rₚₐₜ of the airways of the patient. If the resistance Rᵥₑₙₜ is large compared to the airway resistance Rₚₐₜ, and if the lung compliance C is relatively large, the actual flow W will not fluctuate significantly as a result of model imperfections. However, in order to reduce the power consumption and the required blower pressure, the pressure drop caused in the blower and tubing, equal to the product Rᵥₑᵢₜ · W, will be limited to approx. 5 mbar for any flow rate set-point between 2 liter/min and 90 liter/min. Therefore, resistance Rᵥₑₙₜ is usually made adjustable.

In general, the model of Figs 2 and 3 may not be sufficiently accurate and/or the parameters are not well enough known to guarantee accurate control of the flow rate. Therefore, additional control mechanisms may be required to minimize the errors in the actual flow rate.

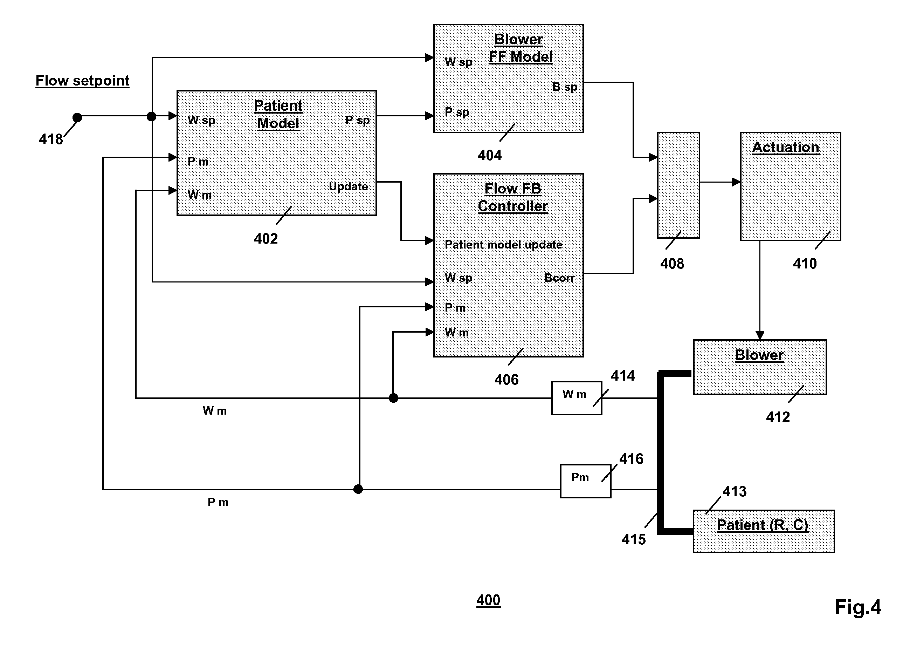

Fig.4 is a block diagram of an embodiment of a control system 400 that takes several of these additional control mechanisms into account. System 400 comprises a patient model module 402, a blower feed-forward model module 404, a flow feedback controller module 406, a combiner module 408, an actuator 410 and a blower 412.

Blower 412 is controlled via a blower actuation module 410. Blower 412 is coupled to a patient 413 via a hose 415. System 400 comprises a sensor 414 for sensing the value Wₘ of the current flow rate, and a sensor 416 for sensing the value Pₘ of the current pressure at blower 412. Sensors 414 and 416 are connected to hose 415. The values Wₘ and Pₘ are supplied to patient model module 402, together with a quantity representative of a flow rate set-point (or: target flow rate value) Wₛₚ at an input 418. Patient model module 402 is based on, e.g., the model discussed above with respect to Figs. 2 and 3. The flow rate set-point Wₛₚ determines a pressure set-point Pₛₚ based on equation (306). Both the values of flow rate set-point Wₛₚ and pressure set-point Pₛₚ are supplied to blower model module 404. Operation of blower model module 404 takes into account the set of fan curves as discussed above with reference to Fig.1, which shows only a single one thereof. As mentioned above, each curve describes the blower's performance for a fixed blower speed. A certain value of pressure set-point Pₛₚ corresponds with a range of values of flow rate set-points, one per curve, i.e., one for each blower speed. The flow rate set-point Wₛₚ received at input 418 determines the relevant curve and therefore the relevant blower speed Bₛₚ. The relevant blower speed Bₛₚ thus determined is supplied to actuation module 410. Actuator 410 converts blower set-point Bₛₚ into a control signal for blower 412. The control signal is, e.g., a speed set-point or a duty-cycle set-point, or the desired value of another physical property representative of the angular speed of the impeller of blower 412. The rotation of the fan causes the flow and, consequently, also a pressure in the ventilation system. Ideally the resulting flow rate Wₘ is exactly equal to the required flow rate set-point Wₛₚ. However, real-life is seldom ideal in this or any other respects, and the value of Wₘ differs from the value of Wₛₚ Therefore, a correction is required that is combined in combiner 408 with the value of blower set-point Bₛₚ in order to improve control of the flow rate.

Module 402 also receives the value Wₘ of the measured flow rate and the value Pₘ of the measured pressure. Using these values and a sequence of previous values of Wₘ and of Pₘ and substituting these in (the numerical equivalent of) equation (306) for each pair of measurements Wₘ and Pₘ individually, enables to extract information about the actual values of parameters R and C in the model used. The actual values of these parameters are characteristic of the individual blower-patient system to be modeled by expression (306). This information is then used to better approximate the actual values of parameters R and C by means of generating updates ΔC and ΔR for these parameters, e.g., through curve fitting or linear regression. The updates are sent to flow-feedback control module 406. It is assumed that the values of ΔC and ΔR are small with respect to the values of C and R, respectively. In addition to the parameter updates ΔR and ΔC, module 406 also receives the value Wₘ of the measured flow rate and the value Pₘ of the measured pressure, and the value of the flow rate set-point Wₛₚ.

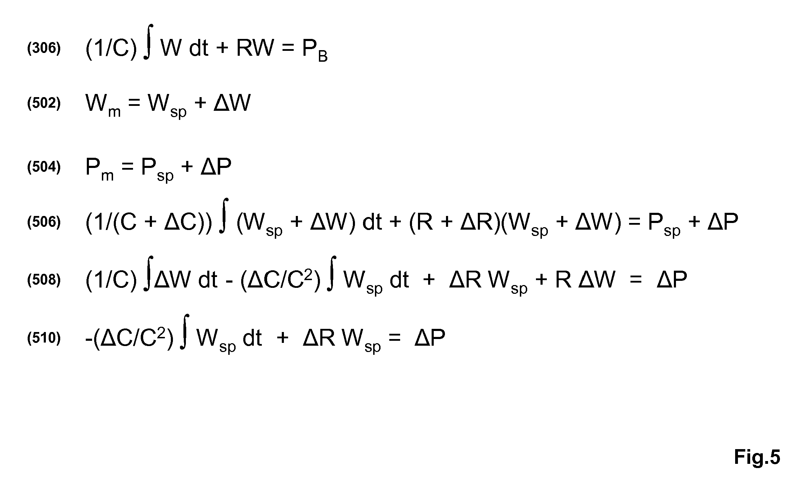

In order to explain operation of module 406, consider equation (306) copied to Fig.5. The value of measured flow rate Wₘ can be thought of as being equal to its set-point value Wₛₚ plus a correction ΔW according to expression (502). The value of measured pressure Pₘ can be thought of as being equal to its set-point value Pₛₚ plus a correction ΔP according to expression (504).

It is assumed that the values of ΔW and ΔP are small compared to the values of Wₛₚ and Pₛₚ, respectively. Substituting the expressions for Wₘ and Pₘ of expressions (502) and (504) into equation (306), together with the corrections ΔR and ΔC, gives expression (506). Next, expression (506) is developed up to the first order of the Δ-quantities, and it is further assumed that Wₛₚ and Pₛₚ comply with expression (306). The result is equation (508).

Now, if flow rate Wₘ is to be equal to flow rate set-point Wₛₚ, then ΔW is to be vanishingly small. Assuming that the terms in expression (508) which comprise the quantity ΔW, can be taken as vanishingly small and can therefore be ignored, then the result is equation (510). Equation (510) expresses the fact that, given the flow rate set-point Wₛₚ, the updates ΔC and ΔR give rise to a change in blower pressure ΔP, and therefore to a correction B_{corr} to blower set-point Bₛₚ. This correction B_{corr} is supplied to combiner 408 and is combined with blower set-point Bₛₚ, so as to adjust the fan speed accordingly.

## Claims

1. Medical equipment comprising a medical ventilator with a blower (412) and a control system (402, 404, 406, 408, 410) for controlling operation of the ventilator, wherein the control system comprises:
an input (418) for receiving a flow rate set-point representative of a first target value of a flow rate;
first means (402) coupled to the input for receipt of the flow rate set-point and configured for determining a pressure set-point, representative of a second target value of a pressure, based on a mathematical model of the ventilator in combination with a patient, the model including a patient parameter indicative of a pneumatic impedance of the patient;
second means (404) coupled to the input for receiving the flow rate set-point and coupled to the first means for receiving the pressure set-point, and configured for determining a blower set-point, based on predetermined fan curves of the blower, for control of the blower.

2. The medical equipment of claim 1, wherein the control system further comprises:
a flow rate sensor (414) for sensing a current flow rate value of a flow rate at an output of the blower;
a pressure sensor (416) for sensing a current pressure value of a pressure at an output of the blower;
third means (402; 406) coupled to the flow rate sensor and the pressure sensor for receiving the current flow rate value and the current pressure value, and configured for determining a correction signal for correction of the blower set-point.

3. The medical equipment of claim 2, wherein the third means is operative to determine the correction signal using at least one of the following strategies: a time delay control algorithm, an iterative learning control algorithm.

4. The medical equipment of claim 3, wherein the control system comprises a selection mechanism for selecting either the time delay control algorithm or the iterative learning control algorithm for use.

5. The medical equipment of claim 1, 2, 3 or 4, wherein the pneumatic impedance comprises at least one of: a resistance of airways of the patient and a compliance of a lung-thorax subsystem of the patient.

6. A control system for controlling operation of medical equipment comprising a medical ventilator with a blower, as specified in claim 1, 2, 3, 4 or 5.
